# EUROPEAN PATENT APPLICATION

(11) **EP 3 936 866 A1**
(43) Date of publication of application: **12.01.2022**
(21) Application number: 20185294.4
(22) Date of filing: 10.07.2020
(51) Int. Cl.: G01N 33/558, G01N 33/569, G01N 33/68

(54) **METHOD OF RAPID DETECTION AND MONITORING OF ANTI SARS-COV-2 ANTIBODIES**

(71) Applicant: Spectral Med Solution AG, 9057 Weissbad (CH)
(72) Inventor: MIEDL, Walter, 9057 Weissbad (CH); KALALI, Behnam Naderi, 80939 München (DE)
(74) Representative: Isarpatent

(57) **Abstract**

The present invention relates to a method of identifying presence of IgM, IgG and IgA antibodies against a target protein or a fragment thereof in whole blood, serum or plasma specimen, by using a solid support which can be a membrane-based cassette. The present invention can be particularly applied to identifying presence of IgM, IgG and IgA antibodies against SARS-CoV-2 or a fragment thereof in whole blood, serum or plasma specimen of a subject. In a single test, presence of said IgM, IgG and IgA antibodies against SARS-CoV-2 or a fragment thereof can be identified in one single solid support by analyzing the regions with different color appearance parameters which appear on the solid support, whereby a broader picture of the infection history of the subject can be visualized.

SEQUENCE LISTING

<110>  Spectral Med Solution AG

<120>  Method of rapid detection and monitoring of anti SARS-CoV-2 antibodies

<130>  P50275-EP

<160>  2

<170>  PatentIn version 3.5

<210>  1
<211>  1273
<212>  PRT
<213>  artificial

<220>
<223>  SARS-CoV-2 S1 protein

<400>  1

<210>  2
<211>  223
<212>  PRT
<213>  artificial

<220>
<223>  RBD of the SARS-CoV2-S1 protein

<400>  2

Arg Val Gln Pro Thr Glu Ser Ile Val Arg Phe Pro Asn Ile Thr Asn
1               5                   10                  15

Leu Cys Pro Phe Gly Glu Val Phe Asn Ala Thr Arg Phe Ala Ser Val
            20                  25                  30

Tyr Ala Trp Asn Arg Lys Arg Ile Ser Asn Cys Val Ala Asp Tyr Ser
        35                  40                  45

Val Leu Tyr Asn Ser Ala Ser Phe Ser Thr Phe Lys Cys Tyr Gly Val
    50                  55                  60

Ser Pro Thr Lys Leu Asn Asp Leu Cys Phe Thr Asn Val Tyr Ala Asp
65                  70                  75                  80

Ser Phe Val Ile Arg Gly Asp Glu Val Arg Gln Ile Ala Pro Gly Gln
                85                  90                  95

Thr Gly Lys Ile Ala Asp Tyr Asn Tyr Lys Leu Pro Asp Asp Phe Thr
            100                 105                 110

Gly Cys Val Ile Ala Trp Asn Ser Asn Asn Leu Asp Ser Lys Val Gly
        115                 120                 125

Gly Asn Tyr Asn Tyr Leu Tyr Arg Leu Phe Arg Lys Ser Asn Leu Lys
    130                 135                 140

Pro Phe Glu Arg Asp Ile Ser Thr Glu Ile Tyr Gln Ala Gly Ser Thr
145                 150                 155                 160

Pro Cys Asn Gly Val Glu Gly Phe Asn Cys Tyr Phe Pro Leu Gln Ser
                165                 170                 175

Tyr Gly Phe Gln Pro Thr Asn Gly Val Gly Tyr Gln Pro Tyr Arg Val
            180                 185                 190

Val Val Leu Ser Phe Glu Leu Leu His Ala Pro Ala Thr Val Cys Gly
        195                 200                 205

Pro Lys Lys Ser Thr Asn Leu Val Lys Asn Lys Cys Val Asn Phe
    210                 215                 220

## Description

### Technical field

The present invention relates to a method of identifying presence of IgM, IgG and IgA antibodies against a target protein or a fragment thereof, particularly the presence of IgM, IgG and IgA antibodies against SARS-CoV-2 or a fragment thereof, in whole blood, serum or plasma specimen, a solid support used in said method, a kit comprising said solid support, and a test result assessment system for performing a colorimetric antibody assay.

### Background

At the end of 2019, the first cases of COVID-19 were identified in Wuhan, China. The virus spread rapidly, infecting tens of thousands of people in China. At a local transmission outside China was detected on 2 February 2020 (Wu D, Wu T, Liu Q, Yang Z. The SARS-CoV-2 outbreak: what we know. International journal of infectious diseases : IJID : official publication of the International Society for Infectious Diseases. 2020). Worldwide there are up to July 10, 2020 already 12,272,098 confirmed cases (Source: CSSE). The existing tests focused on the diagnosis of acute infection using genomic techniques such as PCR-based methods or deep sequencing. These methods for identifying acute infections are useful, but for wide application are only limited suitable. In order to detect an infection, a significant amount of viral genome is needed. This in turn depends strongly on the degree of virus replication at the place of sampling. For this reason, the current tests are designed and developed mostly for identification of symptomatic infections. It is currently estimated that 30% of COVID-19 infections are asymptomatic and up to 85% may be undocumented (H. N, T. K, A. S, S-Mok. J, K. H, R. K, et al. Estimation of the asymptomatic ratio of novel coronavirus infections (COVID-19). International Journal of Infectious Diseases. 2020;https://doi.org/10.1016/j.ijid.2020.03.020., Li R, Pei S, Chen B, Song Y, Zhang T, Yang W, et al. Substantial undocumented infection facilitates the rapid dissemination of novel coronavirus (SARS-CoV2). Science. 2020:eabb3221), therefore, it is essential to have additional testing methods that provide a more complete picture of the infection history of the subjects being tested.

Although RT-qPCR is a highly sensitive test for SARS-CoV-2 (the virus that causes COVID-19), it has several limitations. The accuracy of this method strongly depends on the high quality of the sampling and sufficient amount of viral genome. This is certainly a significant challenge not only because the amount of viral RNA varies tremendously between patients, it is also significantly variable within the same individual depending on the timing of the test and the start of the infection and the onset of symptoms.

Serological tests which analyze the specific antibodies offer some advantages over RT-qPCR:
1) Serological tests detect human antibodies which are known to be much more stable than viral RNA. As a result, antibody serological specimens are less sensitive to spoilage during collection, transport, storage and testing, compared to the RT-qPCR specimens.
2) Unlike RT-qPCR, antibody tests can detect past infection because virus-specific antibodies (unlike viral RNA) persist in the blood for several weeks/months after onset of symptoms.
3) Antibodies are mainly distributed in the blood homogeneously, serological specimens have much less variations than nasopharyngeal viral RNA specimens and can be easily collected with minor phlebotomy discomfort to the patient.

However, in this regard, antibody tests still have several limitations, which are mainly attributed to the slow reaction rate of the human antibody to SARS-CoV-2. Although several studies are still on-going, at present SARS-CoV-2 antibodies are not detectable earlier than 3 days after onset of symptoms (or at least 7 to 10 days after infection).

Document EP 3 470 825 A1 discloses methods and systems for capturing and processing assay test data using a mobile phone.

### Description of the invention

Throughout this description and the following claims, unless the context requires otherwise, the term "comprise", "comprises" or "comprising", should be understood as the inclusion of a stated member, integer or step but not the exclusion of any other non-stated member, integer or step. The term "comprising" encompasses "including" as well as "consisting", for example, a composition "comprising" X may consist exclusively of X or may include something additional, for example, X + Y.

The terms "a" and "an" and "the" in the description should to be understood as being cover both the singular and the plural, unless otherwise indicated herein or clearly contradicted by context. Recitation of ranges of values herein is merely intended to serve as a shorthand method of referring individually to each separate
value falling within the range. Unless otherwise indicated herein, each individual value is incorporated into the description as if it were individually recited herein. No language in the description should be construed as indicating any non-claimed element essential to the practice of the present invention.

The term "and/or" in the present description should be taken as specific disclosure of each of the two specified features or components with or without the other. For example, "A and/or B" is to be taken as specific disclosure of each of (i) A, (ii) B and (iii) A and B, just as if each is set out individually herein.

The first aspect of the present invention relates to a novel point of care rapid method to identify presence of IgM, IgG and IgA antibodies against a target protein or a fragment thereof, particularly to identify presence of IgM, IgG and IgA antibodies against SARS-CoV-2 or a fragment thereof in whole blood, serum or plasma specimen. The method is based on immunological assay and identifies the presence of said IgM, IgG and IgA antibodies in a single test, which comprises the steps of:
(a) *in vitro* contacting a sample of whole blood, serum or plasma specimen of a subject with one reagent zone of a solid support, wherein at least one antigen is present in said reagent zone which binds with the IgM, IgG and IgA antibodies against said target protein or a fragment thereof;
(b) contacting the mixture from step (a) with a first reaction zone in the same solid support wherein the said first reaction zone contains one protein which binds one of said IgM, IgG and IgA antibodies, and a colored region appears in said first reaction zone when said mixture reacts with said one of said IgM, IgG and IgA antibodies.
(c) contacting the mixture after step (b) with a second reaction zone in the same solid support wherein said second reaction zone contains one protein which binds one of said IgM, IgG and IgA antibodies different from that in step (b), and a colored region with at least one different color appearance parameter from that in step (b) appears in said second reaction zone when said mixture after step (b) reacts with said one of IgM, IgG and IgA antibodies different from that in step (b); and
(d) contacting the mixture after step (c) with a third reaction zone in the same solid support wherein said third reaction zone contains one protein which binds one of said IgM, IgG and IgA antibodies different from that in steps (b) and (c), and a colored region with at least one different color appearance parameter from that in steps (b) and (c) appears in said third reaction zone when said mixture after step (c) reacts with said one of IgM, IgG and IgA antibodies different from that in steps (b) and (c).

Preferably, the method of the present invention contains a further *in vitro* step before step (a) wherein said sample of whole blood, serum or plasma specimen of a subject contacts with an additional zone in the same solid support, and wherein said additional zone contains particles conjugated with anti-IgM, anti-IgG and anti-IgA antibodies, more preferably said particles are Cellulose Nano Beads (CNBs).

Preferably, the method of the present invention contains a further *in vitro* step before step (a) wherein said sample of whole blood, serum or plasma specimen of a subject contacts with an additional zone in the same solid support, and wherein said additional zone contains particles conjugated with anti-IgM, anti-IgG and anti-IgA antibodies, and wherein said protein in step (b) is one of IgM, IgG and IgA antibodies, said protein in step (c) is one of IgM, IgG and IgA antibodies different from that in step (b), and said protein in step (d) is one of IgM, IgG and IgA antibodies different from that in steps (b) and (c). More preferably, said particles are Cellulose Nano Beads (CNBs).

Preferably, in the present invention, the anti-IgM, anti-IgG and anti-IgA antibodies independently have an origin of human, nonhuman primates, rabbit, mouse, rat, cow, bird, sheep, goat, cat, dog, pig, or horse cells which are well known for a person skilled in the art, preferably mammal, more preferably human.

Preferably, in the present invention, the anti-IgM, anti-IgG and anti-IgA antibodies are independently selected from anti-human, anti-mouse, anti-rat, anti-sheep, anti-goat, anti-rabbit, anti-donkey, anti-horse antibodies and antibodies derived from other species which are well known for the skilled person in the art.

Preferably, in the present invention, the anti-IgM, anti-IgG and anti-IgA antibodies are independently selected from anti-mouse, anti-rat, anti-sheep, anti-goat, anti-rabbit, anti-donkey, anti-horse antibodies and antibodies derived from other species which are well known for the skilled person in the art.

Preferably, the position order of the additional zone, the reagent zone and the three reaction zones on the solid support is additional zone-reagent zone-three reaction zones.

Preferably, said protein in steps (b), (c) and (d) is one of IgM, IgG and IgA antibodies, more preferably said protein is immobilized on said solid support, and even more preferably said solid support is a membrane or a membrane-based cassette.

Preferably, in the present invention, said solid support is a membrane or a membrane-based cassette, and wherein said membrane is a nitrocellulose membrane.

Preferably, in the present invention, the mixture from step (a) or from said further *in vitro* step before step (a) migrates upward on the membrane by capillary action, whereby to perform from step (a) to step (d), or perform from said *in vitro* step before step (a), and then step (a) to step (d)

Preferably, said proteins in steps (b), (c) and (d) is one of IgM, IgG and IgA antibodies which independently have an origin of human, nonhuman primates, rabbit, mouse, rat, cow, bird, sheep, goat, cat, dog, pig, or horse cells which are well known for a person skilled in the art, preferably mammal, more preferably human.

Preferably, said proteins in steps (b), (c) and (d) is one of IgM, IgG and IgA antibodies are anti species specific antibodies, wherein said species are optionally human, nonhuman primates, rabbit, mouse, rat, cow, bird, sheep, goat, cat, dog, pig, or horse cells which are well known for a person skilled in the art.

Preferably, said protein in steps (b), (c) and (d) is one of human IgM, human IgG and human IgA antibodies.

Preferably, said protein in steps (b), (c) and (d) is one of anti-human IgM, anti-human IgG and anti-human IgA antibodies.

Preferably, the antigen in step (a) is conjugated with or coated on particles. More preferably, said particles are Cellulose Nano Beads (CNBs).

Preferably, the antigen in step (a) is conjugated with or coated on particles, wherein said protein in step (b) is one of anti-IgM, anti-IgG and anti-IgA antibodies, said protein in step (c) is one of anti-IgM, anti-IgG and anti-IgA antibodies different from that in step (b), and said protein in step (d) is one of anti-IgM, anti-IgG and anti-IgA antibodies different from that in steps (b) and (c). More preferably, said particles are Cellulose Nano Beads (CNBs).

Preferably, said proteins in reactions zones in steps (b), (c) and (d) are immobilized on the solid support. More preferably, in this scenario, said solid support is a membrane or a membrane-based cassette.

Preferably, in the present invention, the particles are Cellulose Nano Beads (CNBs), or other Nano particles which are well known for the skilled person in the art.

A second aspect of the present invention relates to an automated test result assessment system, specifically for automatically assessing results obtained by the point of care rapid method according to the first aspect of the invention. The assessment of the test results can advantageously be performed by a layperson, without risks of misinterpretation or mishandling. It may be performed with ubiquitously available and non-expensive hardware components and allows for mobile test result assessment independent from the location of the testing. The test result assessment system may further be built or implemented into a single device or may allow for a modular approach combining several hardware devices and operatively coupling those in mutual communication with each other.

The test result assessment system includes an imaging device that is configured to gather optical images from the various zones of a solid support used to perform a colorimetric assay and to thereby identify the presence of IgM, IgG and IgA antibodies. The solid support may for example be a membrane-based cassette having one or more reagent zones and one or more reaction zones. The membrane of such a cassette may be in the form of a substantially planar strip along the extension of which the various reagent and reaction zones may be spaced apart from each other.

Capturing an image of the reagent zone(s) and reaction zone(s) may advantageously be performed after the sample of whole blood, serum or plasma specimen of a subject has been contacted with the reagent zone and the relevant subsequent mixtures have been contacted with the reaction zones thereafter. After all the necessary and/or desired contacts have been made for the immunological assay, and - in some instances - a certain guard period for allowing the biochemical reactions to happen has elapsed, the image may be captured. In some embodiments of the second aspect, several images may be automatically captured by the imaging device as a sequence along a timeline. The development of the colors in the reagent and/or reaction zones from image to image in the sequence may serve as an indicator when the biochemical reactions happening on the solid support have substantially been finished. It may be possible to automatically track the changes in colors in the sequence of images and to define a cutoff threshold below which a change in colors in the reagent and/or reaction zones indicates that the test result is ready.

In some embodiments of the second aspect, the imaging device may be a separate device, such as a stand-alone camera with a CCD or CMOS imaging sensor, a fluorescence detector, a microscope or any similar device. In other embodiments, the imaging device may be a device integrated into another hardware component, such as for example a CCD or CMOS sensor integrated into a smartphone, a laptop, a tablet, a notebook or any other portable computing device.

The imaging device may be configured to capture all of the various zones of the solid support in a single image to provide identical lighting and image processing conditions for each of the various reagent and reaction zones. In some embodiments of the second aspect, the imaging device may further be configured to capture additional features in the surrounding of the reagent and reaction zones. For example, the imaging device may be configured to detect information attached to the solid support that provides for a unique attribution of the sample with a subject. The attribution information may be in the form of handwritten or machine-generated alphanumerical symbols, QR codes, contactless stickers or other coding schemes for providing an allocation of the sample with a subject, the conditions (such as time, date, place, responsible physician, sample amount) of the test, unique serial numbers for distinction of test among each other and similar background information.

In some embodiments of the second aspect, the imaging device may further be configured to capture reference information associated with the solid support, such as pre-printed coloring schemes on the cassette and/or the membrane strip for calibrating the test results. Moreover, the imaging device may further be configured to capture surrounding imaging conditions such as brightness, contrast, spectral radiance, luminance, luminous exposure and similar photometric parameters. The captured surrounding imaging conditions may also contribute to optimizing the calibration of the imaging information gathered from the reagent and reaction zones.

The test result assessment system further includes an electronic processing device, such as a microcontroller, a microprocessor, an ASIC, an FPGA or any similar computing device, operatively and communicatively coupled to the imaging device. The electronic processing device is configured to receive the capture images from the imaging device for further analysis of the test result.

The test result assessment system may further include a wirebound or wireless communication module that is operatively and communicatively coupled to the electronic processing device. The electronic processing device may send captured images from the imaging device to the communication module for transmission over communication networks, such as for example a network cloud or the Internet, to various other network nodes. For example, the electronic processing device may transmit captured images from the imaging device to a remote storage facility, to a remote central data hub, such as a medical data center, to remote medical facilities such as hospitals, private or governmental medicine institutes, pharmacological research facilities, medical faculties of universities, morgues or quarantine facilities, and/or to remote high-performance computer facilities for extended evaluation capacities.

The electronic processing device may send captured images in raw format, in pre-processed format, in anonymized form for compliance with privacy guidelines, or in fully processed format. It may also be possible in some embodiments that the electronic processing device processes a number of captured images and sends compiled lists, tables or collages of automatic test analyses to the communication module for transmission to remote network nodes.

The test result assessment system may further include a local storage component coupled to the electronic processing device. The local storage component may store an operating system for the electronic processing device, configuration data for the electronic processing device and/or the imaging device and temporarily or permanently captured image data from the imaging device in raw format, pre-processed format, fully processed format and/or condensed or anonymized format.

The test result assessment system may further include an input/output module (IO module) coupled to the electronic processing device. The IO module may be used to present any test results, images in raw, pre-processed, fully processed and/or condensed or anonymized format and/or evaluation data received from one of the remote network nodes via the communication network to a user of the test result assessment system. The electronic processing device may in some embodiments also send or transmit data to be displayed or visualized via the communication module to a communication network, such as the Internet, so that the transmitted data may be output at a website or a software application from a content or provider server connectedly remotely to the communication network. In a similar manner, remote input commands for controlling the test result assessment system may be input via a website or a software application which are then relayed through to the communication network and the communication module to the electronic processing device.

The electronic processing device or any device to which the electronic processing device may transfer the imaging data for further processing via a communication network may employ self-learning algorithms used in a system of artificial intelligence (AI system). In general terms, a self-learning algorithm emulates cognitive functions which, according to human judgement, are assigned to a human cognitive ability. In this case, as a result of new training information being added, the self-learning algorithm can dynamically adapt the knowledge gained heretofore from old training information to the changed circumstances in order to recognize and extrapolate patterns and regularities in the totality of the training information.

In self-learning algorithms within the meaning of the present invention, it is possible to use all kinds of training forming the human acquisition of knowledge, such as, for example, supervised learning, partially supervised learning, independent learning on the basis of generative, non-generative or deep adversarial networks (AN), reinforcement learning or active learning. In this case, feature-based learning ("representation learning") can be used in each instance. The self-learning algorithms within the meaning of the present invention can perform, in particular, an iterative adaptation of parameters and features to be learned by way of feedback analysis.

A self-learning algorithm within the meaning of the present invention can be based on a support vector classifier (SVN), a neural network such as, for instance, a convolutional neural network (CNN), a Kohonen network, a recurrent neural network, a time-delayed neural network (TDNN) or an oscillatory neural network (ONN), a random forest classifier, a decision tree classifier, a Monte Carlo network or a Bayesian classifier. In this case, a self-learning algorithm within the meaning of the present invention can use property-hereditary algorithms, k means algorithms such as, for instance, Lloyd's or MacQueen's algorithms or TD learning algorithms, such as, for instance, SARSA or Q learning.

The self-learning algorithms employed by the electronic processing device and/or a remote processing device remotely connected to the electronic processing device may aid in making the colorimetric assay for determining the colors of the various reagent and/or reaction zones after reaction with the antigens and/or antibodies more resilient to varying image capturing conditions such as varying lighting conditions, varying angles of perspective, varying skew angles and/or varying aging of the chemicals used in the assays during capturing the images with the imaging device.

The test result assessment system may be used to provide a more reliable, consistent and standardized interpretation of the color appearance parameters, such as hue, chroma, colorfulness, lightness and/or brightness, appearing for different testing outcomes in the various reagent and reaction zones of the immunological assay. Moreover, the electronic processing allows for a more comprehensible and safer way for laypersons or medically less well trained testing facility staff to understand the implications of a specific test outcome.

Due to the electronic processing being performed in the test result assessment system, an unbroken chain of certified testing results may be stored in a remote and distributed network environment. When converting the local testing results automatically into standardized digitally available testing data, such testing data may be monitored and certified on-the-fly by means of validation in an entity that is neutral, can be checked and is open at least for the involved parties. A block-chain protocol can be implemented for this purpose in a distributed information network accessible to any test result assessment system via a common communication network. The entries of the block-chain protocol may be concatenated and thus made accessible for checking by the entry-makers. Digitally linking testing results and testing conditions, and the associated possibility for monitoring adherence to medically standardized testing requirements during execution of the immunological assay enable the validation of all testing efforts. The testing results - if considered to be living up to the standards - can be certified in the distributed validation network so that third parties may check whether testing results are actually considered to be valid.

Validating test results of immunological assays in real-time enables monitoring manipulation or forgery of tests, deviations from standardized testing condition ranges, the number of test, the time, date, place and facility of a test and other parameters relating to concerted testing procedures. Such validation methods allow for automatable remote control of large cohort testing, while at the same allowing individual on-site execution of each individual assay. All testing results can be recorded in a validation protocol so as to be safe from manipulation by any parties involved in the process, in accordance with known and specified logging standards. If required, certificates regarding the proven quality of the test results can be issued on the basis of this validation protocol, the authenticity of which certificates can also be easily checked by third parties.

The complete digitisation of the validation and certification process reduces the logistical and administrative effort to a minimum. Furthermore, the paperless documentation chain allows all parties involved to save on costs. The distributed system makes archiving of the validation and certification history extremely efficient and secure from data loss. Moreover, having data centralized available in real-time enables a faster access to test results of larger cohorts of tested subjects so that political and epidemiological decisions may be made much faster when reliable and verifiable results are available in real-time or at least near real-time.

Preferably, in the present invention the term "fragment" refers to an analogue of the corresponding protein.

Preferably, the above-disclosed fragments comprise an amino acid sequence which shows an amino acid identity of more than 70%, preferably more than 75%, more preferably more than 80% to the full-length of the corresponding protein, or shows an amino acid identity of 80%, 81%, 82%, 83%, 84%, 85%, 86%, 87%, 88%, 89%, 90%, 91%, 92%, 93%, 94%, 95%, 96%, 97%, 98%, 99% or 100% to the full-length of the corresponding protein.

Preferably, the term "fragment" refers to a polypeptide comprising the one or more of the conserved domains of the corresponding protein.

Preferably, the term "fragment" refers to a polypeptide comprising at least a region which shows an amino acid identity of more than 70%, preferably more than 75%, more preferably more than 80% to one of the conserved domains of the corresponding protein, or shows an amino acid identity of 81%, 82%, 83%, 84%, 85%, 86%, 87%, 88%, 89%, 90%, 91%, 92%, 93%, 94%, 95%, 96%, 97%, 98%, 99% or 100% identical to one of the conserved domains of the corresponding protein.

Preferably, in the composition disclosed above, one or more of proteins or fragments thereof included therein have an origin of vertebrate, e.g. human, nonhuman primates, rabbit, mouse, rat, cow, bird, sheep, goat, cat, dog, pig, or horse cells, preferably mammal, more preferably human.

In the present invention, the term "antibody", particularly the "antibody" conjugated in the different reaction zones of the solid support, encompasses various forms of antibodies, preferably monoclonal antibodies including, but not being limited to, whole antibodies, antibody fragments, human antibodies, chimeric antibodies, humanized antibodies and genetically engineered antibodies (variant or mutant antibodies) as long as the characteristic properties according to the invention are retained. Human or humanized monoclonal antibodies and recombinant antibodies, in particular recombinant monoclonal antibodies, are preferred. Thus, the antibody, according to the present invention is preferably a monoclonal antibody. Moreover, it is also preferred that the antibody is a multi-chain antibody, i.e. an antibody comprising more than one chain, which is thus different from a single-chain antibody.

In the present invention, said method can be particularly applied to test presence of IgM, IgG and IgA antibodies against SARS-CoV-2 in in whole blood, serum or plasma specimen. Preferably, said IgM, IgG and IgA antibodies are detected against SARS-CoV-2 S1 protein which has a sequence of SEQ ID NO: 1:

"

















"

Preferably, said IgM, IgG and IgA antibodies detected against SARS-CoV-2 S1 protein are also detected against Receptor Binding Domain (RBD) of SARS-CoV-2 protein.

As used herein, the term "antigen" refers to any structural substance which serves as a target for the receptors of an adaptive immune response, in particular as a target for antibodies, T cell receptors, and/or B cell receptors. The used part of the S1 protein of SAR-CoV2 binds to ACE2 receptor of the human cells. Preferably, in the present invention, this RBD (Receptor Binding Domain) which is part of the S1 is used as antigen. An "epitope", also known as "antigenic determinant", is the part (or fragment) of an antigen that is recognized by the immune system, in particular by antibodies, T cell receptors, and/or B cell receptors. Thus, one antigen has at least one epitope, i.e. a single antigen has one or more epitopes. An antigen may be (i) a peptide, a polypeptide, or a protein, (ii) a polysaccharide, (iii) a lipid, (iv) a lipoprotein or a lipopeptide, (v) a glycolipid, (vi) a nucleic acid, or (vii) a small molecule drug or a toxin. Thus, an antigen may be a peptide, a protein, a polysaccharide, a lipid, a combination thereof including lipoproteins and glycolipids, a nucleic acid (e.g. DNA, siRNA, shRNA, antisense oligonucleotides, decoy DNA, plasmid), or a small molecule drug (e.g. cyclosporine A, paclitaxel, doxorubicin, methotrexate, 5-aminolevulinic acid), or any combination thereof. Preferably, the antigen is selected from (i) a peptide, a polypeptide, or a protein, (ii) a polysaccharide, (iii) a lipid, (iv) a lipoprotein or a lipopeptide and (v) a glycolipid; more preferably, the antigen is a peptide, a polypeptide, or a protein.

Preferably, the solid support is a membrane or a membrane-based cassette, wherein more preferably, the antigen in the reagent zone is immobilized on the membrane when particles are present in the additional zone and the anti-IgM, anti-IgG and anti-IgA antibodies are conjugated on said particles as disclosed above, or the antigen is conjugated on the particles in the reagent zone as disclosed above, and wherein even more preferably said particles in above two scenarios are CNBs. For testing the presence of IgM, IgG and IgA antibodies against SARS-CoV-2 or a fragment thereof in whole blood, serum or plasma specimen, to guarantee the specificity of the assay, a comprehensive literature research and bioinformatics analysis was applied. Accordingly, to serve as an antigen, we choose a part of the S1 protein of the spike of the SARS-CoV-2 which is responsible to bind to the host receptor. This so called RBD protein (Receptor Binding Domain) is 100% for the COVID-19 virus. The RBD of the SARS-CoV2-S1 protein having the sequence of SEQ ID NO: 2:

“RVQPTESIVRFPNITNLCPFGEVFNATRFASVYAWNRKRISNCVADYSVLYNSASFSTFKCYGVSPTKLNDL
CFTNVYADSFVIRGDEVRQIAPGQTGKIADYNYKLPDDFTGCVIAWNSNNLDSKVGGNYNYLYRLFRKSNL
KPFERDISTEIYQAGSTPCNGVEGFNCYFPLQSYGFQPTNGVGYQPYRVVVLSFELLHAPATVCGPKKSTNLV
KNKCVNF”.

Preferably, other domains of SARS-CoV-2, such as Nucleoprotein N, can also be used alone or in combination to serve as an antigen.

The RBD can be produced in eukaryotic cells as recombinant protein. It can be uses as biomarker as antigen for different immunological assays.

Preferably, one or more antigens are conjugated in the reagent zone of the solid support. More preferably, one or more antigens are conjugated with a determined concentration in the reagent zone of the solid support. Even more preferably, there are more than one reagent zone in the solid support.

Preferably, the amount of the sample of whole blood, serum or plasma specimen of a subject is 10 µl to 1 ml, more preferably 20 µl to 500 µl even more preferably 40 µl to 200 µl. Said amount of the sample can also refer to a volume after the sample is diluted with a buffer.

Preferably, the solid support is a membrane or membrane-based cassette, and wherein the mixture from step (a) migrates upward on the membrane by capillary action. More preferably, the membrane is in a form of a strip, and the reagent zone is located near one end of the membrane strip, wherein after reacting with the antigen in said reagent zone the sample of whole blood, serum or plasma specimen of a subject diffuses continuously toward the other end of the membrane strip. Further preferably, the three reaction zones are located separately from lower middle region, which is close to the reagent zone, to the upper middle region of the membrane, which is close to the other end of the membrane.

Preferably, the solid support contains three or more reaction zones, in addition the one or more reagent zones.

Preferably, the color regions are line-shaped. Those lines may preferably differ in at least the color appearance parameter of color hue, more preferably in that the three different color hues said three line-shaped color regions which appear in the solid support are green, blue and red. Preferably, the three lines on the membrane are respectively human IgA, IgM, IgG or anti species specific antibodies immobilized on the solid phase. More preferably, the solid phase is a membrane. After binding of respective conjugated anti-human antibodies they become colored and visible. More preferably, said anti-IgM, anti-IgG and anti-IgA antibodies are conjugated with CNB. They guarantee the technical validity of the test procedure and serve as reference signal for calibration of the sensor. The test is considered and reported as invalid if any of these control calibration lines does not appear.

Color appearance parameters describe the degree to which a visual stimulus can be described as similar to or different from visual stimuli in terms of various perception distinctions. Different color appearance parameters may be color hue, chroma (or saturation), colorfulness, intensity, brightness and lightness. Color hue as the most important distinction for colorimetric assays may typically be given a single number value representing an angular position of the perceived stimulus around a reference axis in a two-dimensional colorspace plot, for example a chromaticity diagram or a color wheel. Preferably, the generation of the color is attributed at least partially to the presence of particles in the present invention. The mechanism of the generation of the color is known to the person skilled in the art, based on the disclosure of the present invention, as well as in the light of common general knowledge.

Preferably, IgM, IgG and IgA antibodies respectively immobilized on the three reactions zones of the solid support are human IgM, human IgG and human IgA antibodies respectively. More preferably, said solid support is a membrane or a membrane-based cassette and said IgM, IgG and IgA or species specific antibodies like anti-goat, anti-mouse, anti-rabbit or similar antibodies are immobilized on the membrane. The term "human antibody", as used herein, is intended to include antibodies having variable and constant regions derived from human immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 3340). Human antibodies can also be produced in phage display libraries (Hoogenboom, H. R., and Winter, G., J. Mol. Biol. 227 (1992) 381-388; Marks, J. D., et al., J. Mol. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). The term "human antibody" as used herein also comprises such antibodies which are modified, e.g. in the variable region, to generate the properties according to the invention.

The second aspect of the present invention is to provide a solid support for identifying presence of IgM, IgG and IgA antibodies against a target protein or a fragment thereof in whole blood, serum or plasma specimen, which comprises a reagent zone containing at least one antigen which binds to the IgM, IgG and IgA antibodies against said target protein or a fragment thereof, characterized in that: said solid support comprises three reaction zones respectively conjugated with IgM, IgG and IgA antibodies, wherein a colored line appears in each reaction zone when the CNB-conjugated- anti-IgM, anti-IgG or anti-IgA antibodies respectively captures the IgM, IgG or IgA antibody against said target protein or a fragment thereof in said whole blood, serum or plasma specimen, and wherein each colored line exhibits a different color from that of the other two colored lines. Preferably, the three antibodies IgA, IgM and IgG detected against said target protein or a fragment thereof may bind to the antigen simultaneously. The presence and stringency of the respective color signal giving by the mentioned antibody complex can be assessed using the above described sensor and computational aids.

The solid support might contain one or more reagent zones. Preferably, the solid support contains three or more reaction zones, in addition to the one or more reagent zones.

Preferably the solid support is a membrane-based cassette, and wherein a liquid mixture containing said whole blood, serum or plasma specimen can migrate upward on the membrane by capillary action. More preferably, the membrane is in a form of a strip, wherein the reagent zone can be located near one end of the membrane strip. Even more preferably, the three reaction zones are located separately from lower middle region, which is close to the reagent zone, to the upper middle region of the membrane, which is close to the other end of the membrane.

Preferably, the antigen in said reagent zone in the solid support comprises a part of the S1 protein of the spike of the SARS-CoV-2.

Preferably, the antigen comprises RBD (Receptor Binding Domain) of the SARS-CoV2- S1 protein which has a sequence of SEQ ID NO:2:

“RVQPTESIVRFPNITNLCPFGEVFNATRFASVYAWNRKRISNCVADYSVLYNSASFSTFKCYGVSPTKLNDL
CFTNVYADSFVIRGDEVRQIAPGQTGKIADYNYKLPDDFTGCVIAWNSNNLDSKVGGNYNYLYRLFRKSNL
KPFERDISTEIYQAGSTPCNGVEGFNCYFPLQSYGFQPTNGVGYQPYRVVVLSFELLHAPATVCGPKKSTNLV
KNKCVNF”.

Preferably, said antigen comprises the sequence of one or more other domains of SARS-CoV-2, such as Nucleoprotein N. More preferably, said antigen comprises one of RBD and Glycoprotein N, or both of them.

Preferably, IgM, IgG and IgA antibodies are respectively conjugated on the three reactions zones of the solid support, wherein said solid support is preferably a membrane and said antibodies are immobilized on said membrane. More preferably, the three different colors of said three colored lines which appear in the solid support are green, blue and red. Preferably, the conjugation pad includes the anti-human IgA, anti-human IgG and anti-human IgM each conjugated with CNBs with different colors, such as Green, Red and Blue. These conjugated antibodies may bind to the FC part of their respective antibodies in the sample. In case of presence of anti SARS-CoV-2 antibodies in the sample, the complex of human antibody plus CNB conjugated anti-human antibody may bind to the antigen in the reagent zone. The color and stringency of the signal at the test line may correlated to the titer of antibodies in the sample. For instance, if anti-human IgG is conjugated with blue CNB, the blue color of the test line is more intensive in a sample with high titer of anti-SARS-CoV-2 IgG is more intensive than a sample with lower titer of this antibody. Preferably, the unbound (excess) of CNB conjugated antibodies will bind to the control lines which are immobilized human antibodies.

To evaluate the specificity of the antigen and proof the concept, ELISAs based on sandwich procedure for each IgM, IgG and IgA can be established. The RBD can be coated in the ELISA plate. The antigen specific antibodies could bind to the antigen. Subsequently, the presence of the anti- SARS-CoV-2 antibodies in the sample can be detected by adding of anti-human IgM, IgG or IgA antibodies which were conjugated with HRP (Horse Radish Peroxidase).

The third aspect of the present invention is to provide a kit for identifying presence of IgM, IgG and IgA antibodies against a target protein or a fragment thereof in whole blood, serum or plasma specimen, comprising the solid support disclosed in the present invention, wherein said kit further comprises a buffer and/or a dropper which facilities the binding of the antigen with the IgM, IgG and IgA antibodies against said target protein or a fragment thereof in the reagent zone of the solid support.

### Figures

The present invention will be described in greater detail below with reference to the embodiments shown in the schematic drawings, in which:
Fig. 1 shows the schematic construction of the test.
Fig. 2 shows test results of an anti-SARS-CoV-2 antibody positive serum sample and a negative serum sample.
Fig. 3 shows test results of two anti-SARS-CoV-2 antibody positive samples (#001 and #002) and a negative whole blood sample.
Fig. 4 shows illustrative stages of an assessment procedure of the test results.
Fig. 5 schematically illustrates a block diagram of components of a test result assessment system.

### Examples

The present invention will now be described in detail with reference to examples thereof. However, these examples are illustrative and do not limit the scope of the invention.

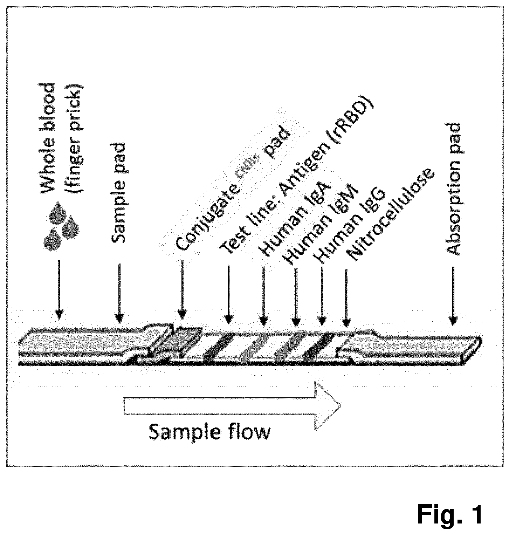

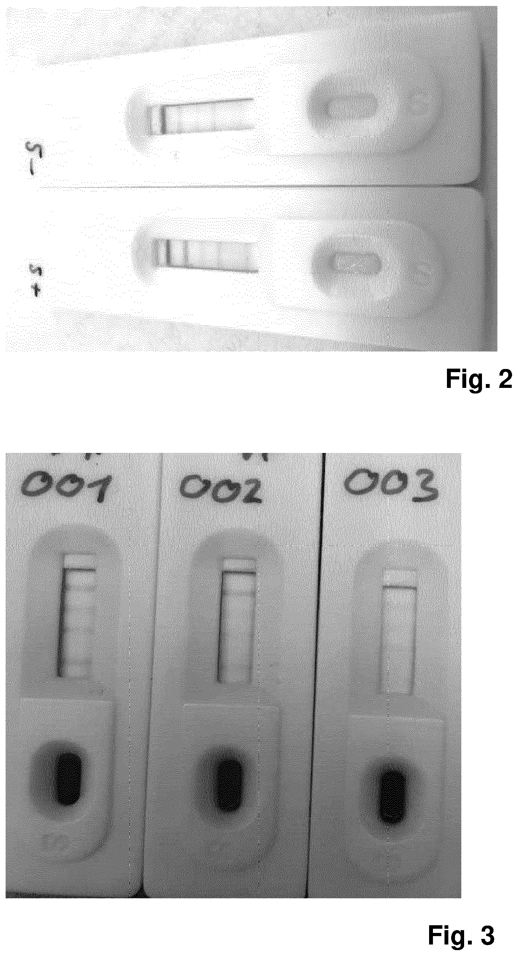

An anti-SARS-CoV-2 antibody positive serum sample and a negative serum sample can be tested through the method of the present invention. Control lines are to calibrate the image processing. The solid support is a membrane-based cassette. An additional zone is present in said solid support which comprise CNBs conjugated with anti-IgG, anti-IgM, and anti-IgA antibodies (Fig. 1). The Test line corresponds to the reagent zone disclosed above comprising the antigen which is the RBD as disclosed above (Fig. 1). Blue line represents IgG, red is for IgM and green line represents the IgA (Fig. 1). During test, the test line contains a combination of target IgG, IgM and IgA SARS-CoV-2 antibodies with different proportions (Fig. 1). Similarly, SARS-CoV-2 antibody positive and negative whole blood samples can also be tested through the method of the present invention (Fig. 2 and 3).

To evaluate the specificity of the antigen and proof the concept, ELISAs based on sandwich procedure for each IgM, IgG and IgA were established. Briefly, the RBD was coated in the ELISA plate. The antigen specific antibodies could bind to the antigen. Subsequently, the presence of the anti- SARS-CoV-2 antibodies in the sample were detected by adding of anti-human IgM, IgG or IgA antibodies which were conjugated with HRP (Horse Radish Peroxidase). Analysis of more than 100 samples in this set of experiments revealed an absolute specificity of the antigen for anti-SARS-CoV-2 antibodies.

Furthermore, in the present invention we have utilized an ICT based method to use imaging and AI technologies to simplify analysis of sample tests in order to give more intuitive and accurate results to users with additional assistants from clinicians and specialists (Fig. 3).

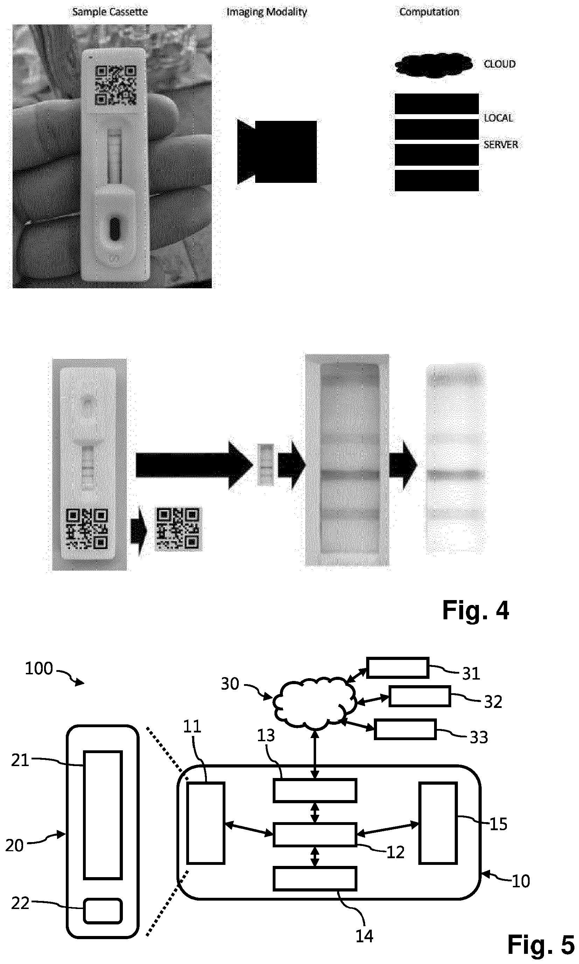

Fig. 4 is a schematic illustration of a test result assessment system 100 that may be used to automatically or at least semi-automatically analyze test results of the serological assay.

The test result assessment system 100 includes an imaging device 11 that is configured to gather optical images from the various zones 21 of a solid support 20 used to perform a colorimetric assay and to thereby identify the presence of IgM, IgG and IgA antibodies against SARS-CoV-2. The solid support 20 may for example be a membrane-based cassette having one or more reagent zones and one or more reaction zones.

The imaging device 11 may for example be a module integrated into another hardware component, such as for example a CCD or CMOS sensor integrated into a smartphone, a laptop, a tablet, a notebook or any other portable computing device 10. Alternatively, the imaging device 11 may also be a separate device, such as a stand-alone camera with a CCD or CMOS imaging sensor, a fluorescence detector, a microscope or any similar device.

The imaging device 11 can capture all of the various zones 21 of the solid support in a single image to provide identical lighting and image processing conditions for each of the various reagent and reaction zones. The imaging device 11 may also capture additional features in the surrounding of the reagent and reaction zones 21, such as for example information attached to the solid support that provides for a unique attribution of the sample with a subject. Exemplarily a QR code 22. The QR code 22 may include information about an allocation of the sample with a subject, the conditions (such as time, date, place, responsible physician, sample amount) of the test, unique serial numbers for distinction of test among each other and similar background information.

The portable computing device 10 further includes an electronic processing device 12, such as a microcontroller, a microprocessor, an ASIC, an FPGA or any similar computing device, operatively and communicatively coupled to the imaging device 11. The electronic processing device 12 is configured to receive the capture images from the imaging device 11 for further analysis of the test result.

A wirebound or wireless communication module 13 is operatively and communicatively coupled to the electronic processing device 12. The electronic processing device 12 may send captured images from the imaging device 11 to the communication module 13 for transmission over communication networks 30, such as for example a network cloud or the Internet, to various other network nodes 31, 32, 33. For example, the electronic processing device 12 may transmit captured images from the imaging device 11 to a remote storage facility, to a remote central data hub 31, such as a medical data center, to remote medical facilities 32 such as hospitals, private or governmental medicine institutes, pharmacological research facilities, medical faculties of universities, morgues or quarantine facilities, and/or to remote high-performance computer facilities 33 for extended evaluation capacities.

A local storage component 14 coupled to the electronic processing device 12 stores an operating system for the electronic processing device 12, configuration data for the electronic processing device 12 and/or the imaging device 11 and temporarily or permanently captured image data from the imaging device 11 in raw format, pre-processed format, fully processed format and/or condensed or anonymized format.

An input/output module (IO module) 15 coupled to the electronic processing device 12 such as a display, is configured to output data prepared by the electronic processing device 12 and/or evaluation data received from one of the remote network nodes 31, 32, 33 via the communication network 30 to a user of the portable computing device 10.

The electronic processing device 12 may in some instances additionally comprise an AI system. The AI system may for example include an AI processor, a rule set generator based on self-learning algorithms, and a reference rule set memory. The AI system may for example initially be fed with a plurality of annotated observation data regarding images taken of solid supports when contacted with serum samples of known origin and composition, in particular with known immunological status. This annotated observation data can serve as a basis for the detection of patterns regarding possible outcomes of the serological assay by means of the rule set generator. The rule set generator can comprise for example a random forest classifier, a support vector classifier, a neural network, a decision tree classifier, a Bayesian classifier or a Monte Carlo network.

At first, the detected patterns regarding possible outcomes of the serological assay are stored iteratively in a training rule set to be constantly and dynamically being updated. An operative reference rule set is formed from the training rule set and is stored in the reference rule set memory by the rule set generator. Checking newly taken images for colorometric patterns that are to be expected involves the AI processor 4 comparing relevant content of the images to be checked with reference patterns in one or more reference rule sets stored in the reference rule set memory. Depending on the deviations or similarities to the one or more reference rule sets, the AI processor may output results of the comparison analysis. The rule set generator may concomitantly update any reference rule sets stored in the reference rule set memory at periodic intervals on the basis of newly added observation data or on the basis of new external predefinitions.

The AI processor may for example output one of a plurality of predefined indicator signals to the electronic processing device 12 for further processing and for outputting a test result assessment signal to the IO module 15 and/or to the communication network 30 for further routing to remote network nodes 31, 32, 33. The test result assessment signal may be based on the received indicator signal of the AI processor in order to convey the determined test result to a user of the test result assessment system 100.

Any test results assessed by the test result assessment system 100 may be transmitted to a distributed validation network where said data are validated, i.e. checked for consistency and for compatibility with previously defined testing conditions. In particular, validating the test results allow for tracing the tests over time, date, place, testing staff, subject being tested and compatibility with the testing requirements as defined by certain testing standards issued by testing organizations or governmental bodies. The test result data is supplemented with a cryptographically encoded checksum, for example a hash function value. The hash function used may include the transmitted test result data and the cryptographically encoded checksum of the last log entry of a test history log, i.e. a block-chain protocol of all tests previously performed, as the function argument. The supplemented test result data is then added to the print history log managed by the distributed validation network. This test history log (sometimes also called block-chain) can be accessed by all participating nodes in the distributed validation network, for example through regularly and periodically performed flooding. The most recent test history log having correct entries is used for further processing, i.e. concatenating further test result entries to the log. The cryptographically encoded checksums included in the blocks of the block-chain may serve as a proof for validity of all previous entries.

Certification entities in the distributed validation network may be used to create a certificate for each test result validly included in the test history log to successful validation of the performed test to third entities. Certificates of this kind are issued only if the test results have been released by the distributed validation network as being valid and performed in line with the testing requirements. In this case, the certificate can be added, together with a cryptographically encoded checksum, to the test history log, for example again using a hash protocol.

The lack of deeper knowledge about the development of SARS-CoV-2 specific immunity and in particular the definition of protective immune responses prevent currently investigating and identifying those persons who may already be protected from infection. The present invention provides serological tests which could give a better picture of the entire infection rate, including subclinical infections, and possibly strategies for guide risk stratification by identifying persons with previous infection who are less vulnerable. This would have far-reaching implications for the functionality of the health care and social life in Germany and worldwide.

One application of the method of the present invention is testing the health state of a subject with regard to COVID-19. In this regard, the analysis of antibody responses and dissection of the class of the expressed antibodies alone, or in combination with RT-qPCR is a valuable diagnostic tool for examining the health state of the subject with regard to COVID-19 (see Table 1). The serological assay in the present invention provides a fast test wherein the presence of IgM, IgG and IgA antibodies against SARS-CoV-2 can be tested at the same time in one single set. Therefore, the present invention can be used in high-throughput screening of the whole population. The method of the present invention enables assessing the overall immune responses to the virus and meanwhile identifying the asymptomatic as well as symptomatic individuals with COVID-19. Profile of detectable antibodies against RBD can provide a broader assessment with regard to the situation of the disease. The table below briefly depicts the rational between antibody test results and clinical significance.

**Table 1**

| **Test results** | | | | **Clinical significance** |
|---|---|---|---|---|
| **RT-PCR** | **IgM** | **IgG** | **IgA** | |
| + | - | - | - | The window period of infection the RT-PCR is false-positive |
| + | + | - | - | Early stage if infection |
| + | + | + | + | Active phase of infection with mucosal immune reaction |
| + | + | + | - | Active phase of infection w/o mucosal immune reaction |
| + | - | + | + | The late or recurrent stage of infection with mucosal immune reaction |
| + | - | + | - | The late or recurrent stage of infection w/o mucosal immune reaction |
| - | + | + | + | The recovery stage of an infection or the RT-PCR is false-negative |
| - | - | + | + | Past infection/recovered with mucosal immune reaction |
| - | + | - | - | Early stage of infection or the RT-PCR is false-negative |

The combination of a **multiplex** lateral flow assay to detect **specifically at the same time** all three **IgM, IgG and IgA** antibodies, particularly anti-SARS-CoV-2 antibodies, is one major contribution which the present invention makes over the prior art. Particularly, the method in the present invention can also comprise the assessment and evaluation of the results using **image processing** and **artificial intelligence,** which further renders the present invention superior than the conventional method. It has to be mentioned that until now- according to our knowledge-there is no other test offering all of above stated advantages, neither for COVID-19 nor for any other diagnostic purpose.

## Claims

1. A method of identifying presence of IgM, IgG and IgA antibodies against a target protein or a fragment thereof in whole blood, serum or plasma specimen, comprising the steps of:
(a) *in vitro* contacting a sample of whole blood, serum or plasma specimen of a subject with one reagent zone of a solid support, wherein at least one antigen is present in said reagent zone which binds with the IgM, IgG and IgA antibodies against said target protein or a fragment thereof;
(b) contacting the mixture from step (a) with a first reaction zone in the same solid support wherein the said first reaction zone contains one protein which binds one of said IgM, IgG and IgA antibodies, and a colored region appears in said first reaction zone when said mixture reacts with said one of said IgM, IgG and IgA antibodies.
(c) contacting the mixture after step (b) with a second reaction zone in the same solid support wherein said second reaction zone contains one protein which binds one of said IgM, IgG and IgA antibodies different from that in step (b), and a colored region with at least one different color appearance parameter from that in step (b) appears in said second reaction zone when said mixture after step (b) reacts with said one of IgM, IgG and IgA antibodies different from that in step (b); and
(d) contacting the mixture after step (c) with a third reaction zone in the same solid support wherein said third reaction zone contains one protein which binds one of said IgM, IgG and IgA antibodies different from that in steps (b) and (c), and a colored region with at least one different color appearance parameter from that in steps (b) and (c) appears in said third reaction zone when said mixture after step (c) reacts with said one of IgM, IgG and IgA antibodies different from that in steps (b) and (c).

2. The method of claim 1, further comprising an *in vitro* step before step (a) wherein said sample of whole blood, serum or plasma specimen of a subject contacts with an additional zone in the same solid support, and wherein said additional zone contains particles conjugated with anti-IgM, anti-IgG and anti-IgA antibodies, and wherein said protein in step (b) is one of IgM, IgG and IgA antibodies, said protein in step (c) is one of IgM, IgG and IgA antibodies different from that in step (b), and said protein in step (d) is one of IgM, IgG and IgA antibodies different from that in steps (b) and (c).

3. The method of claim 1 or 2, wherein the target protein is S1 protein of SARS-CoV-2 with a sequence of SEQ ID NO: 1.

4. The method of any one of claims 1 to 3, wherein the antigen in said reagent zone of the solid support is a part of the S1 protein of the spike of the SARS-CoV-2 with a sequence of SEQ ID NO: 2.

5. The method of any one of claims 1 to 4, wherein the solid support is a membrane or a membrane-based cassette, and wherein the mixture from step (a) or from said *in vitro* step before step (a) migrates upward on the membrane by capillary action, whereby to perform from step (a) to step (d), or perform from said *in vitro* step before step (a), and then step (a) to step (d).

6. The method of any one of claims 1 to 5, wherein the colored regions are line-shaped and differing in hue, and optionally wherein the three different color hues are green, blue and red.

7. The method of any one of claims 1 to 6, further comprising the steps of:
(e) electronically capturing one or more images of at least the reagent zone and the first, second and third reactions zones; and
(f) electronically processing the one or more captured images with an artificial intelligence system employing self-learning algorithms to determine at least one color appearance parameter, in particular the color hue, of the differently colored regions in the first, second and third reactions zones.

8. The method of claim 7, further comprising the steps of:
(g) transferring an information file comprising the determined color hues and determined testing conditions of the performed assay to a distributed validation network; and
(h) validating the transferred information file by means of the distributed validation network; and
(i) adding the determined color appearance parameters and determined testing conditions of the performed assay, together with a cryptographically encoded checksum, to a testing history block-chain based protocol managed by the distributed validation network.

9. The method of claim 7 or 8, wherein the one or more captured images are electronically processed with an artificial intelligence system executed on a mobile device that includes an imaging device with which the one or more images have been captured.

10. The method of claim 7 or 8, wherein the one or more captured images are transferred to a remote communication network and are electronically processed with an artificial intelligence system executed on a remote evaluation device separate from the imaging device with which the one or more images have been captured.

11. A solid support for identifying presence of IgM, IgG and IgA antibodies against a target protein or a fragment thereof in whole blood, serum or plasma specimen, which comprises a reagent zone containing at least one antigen which binds with the IgM, IgG and IgA antibodies against said target protein or a fragment thereof, **characterized in that**: said solid support further comprises three reaction zones respectively immobilized with IgM, IgG and IgA antibodies, wherein a colored line appears in each reaction zone when the anti-IgM, anti-IgG or anti-IgA antibody respectively captures the IgM, IgG or IgA antibody against said target protein or a fragment thereof in said whole blood, serum or plasma specimen, and wherein each colored line exhibits a different color from that of the other two colored lines, preferably said colored lines are green, blue and red.

12. The solid support of claim 11, wherein the solid support comprises an additional zone which comprises particles conjugated with anti-IgM, anti-IgG and anti-IgA antibodies.

13. The solid support of claim 11 or 12, wherein the solid support is a membrane or a membrane-based cassette, and wherein a liquid mixture containing said whole blood, serum or plasma specimen can migrate upward on the membrane by capillary action.

14. The solid support of any one of claims 11 to 13, wherein the antigen in said reagent zone is a part of the S1 protein of the spike of the SARS-CoV-2 with a sequence of SEQ ID NO: 2.

15. A kit for identifying presence of IgM, IgG and IgA antibodies against a target protein or a fragment thereof in whole blood, serum or plasma specimen, comprising the solid support of any one of claims 11 to 14, wherein said kit further comprises a buffer and/or a dropper which optionally facilities the binding of the antigen with the IgM, IgG and IgA antibodies against said target protein or a fragment thereof in the reagent zone of the solid support.
